(19) Europäisches Patentamt
European Patent Office
Office européen des brevets

(11) Numéro de publication: **0 037 116**
**B1**

(12) **FASCICULE DE BREVET EUROPEEN**

(45) Date de publication du fascicule du brevet: **14.12.83**

(21) Numéro de dépôt: **81102399.3**

(22) Date de dépôt: **30.03.81**

(51) Int. Cl.³: **C 07 C 13/32**, C 07 C 1/253, C 07 C 1/32, C 07 C 5/05, C 07 C 147/02

(54) **Composés bicycliques polyinsaturés, leur préparation et leur utilisation à titre de produits de départ pour la préparation de composés bicycliques monoinsaturés.**

(30) Priorité: **31.03.80 CH 2535/80**

(43) Date de publication de la demande:
**07.10.81 Bulletin 81/40**

(45) Mention de la délivrance du brevet:
**14.12.83 Bulletin 83/50**

(84) Etats contractants désignés:
**CH DE FR GB LI NL**

(56) Documents cités:
EP - A - 0 001 873
DE - A - 2 916 418
US - A - 3 360 577
US - A - 3 949 011

Chemical Abstracts vol. 52, no. 6, 1958
Columbus, Ohio, USA K. BIEMANN et al.
"Structure and synthesis of muscopyridine"
colonnes 4635 d à 4637 b

(73) Titulaire: **FIRMENICH SA**
**Case postale 239**
**CH-1211 Genève 8 (CH)**

(72) Inventeur: **Fehr, Charles**
**16 bis, chemin des Genêts**
**CH-1202 Geneve (CH)**

(74) Mandataire: **Schmied-Kowarzik, Volker, Dr. et al,**
**Siegfriedstrasse 8**
**D-8000 München 40 (DE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Composés bicycliques polyinsaturés, leur préparation et leur utilisation à titre de produits de départ pour la préparation de composés bicycliques monoinsaturés

L'invention se rapporte à des composés bicycliques polyinsaturés nouveaux, utiles à titre de produits intermédiaires dans la synthèse de cétones macrocycliques odorantes, EXALTONE® et muscone plus particulièrement.

L'invention a plus précisément pour objet des composés de formule

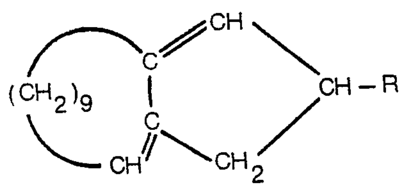

(III)

dans laquelle le symbole R représente un atome d'hydrogène ou un radical méthyle.

Elle a également pour objet un procédé pour la préparation desdits composés (III), caractérisé en ce qu'on traite au moyen d'une base forte un composé de formule

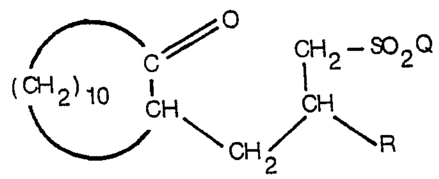

(II)

dans laquelle le symbole R est défini comme ci-dessus et Q représente un radical alkyle ou aryle.

L'invention a en outre pour objet l'utilisation desdits composés (III) à titre de produits de départ pour la préparation d'un composé de formule

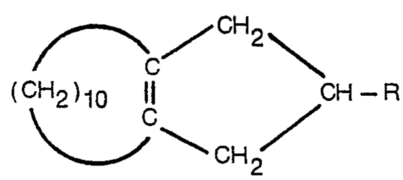

(I)

dans laquelle le symbole R représente un atome d'hydrogène ou un radical méthyle, caractérisée en ce que l'on hydrogène ledit composé de formule (III) en présence d'un catalyseur métallique.

Elle a finalement pour objet les composés de formule

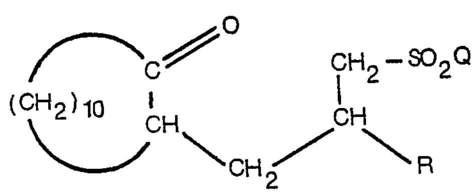

(II)

dans laquelle le symbole R représente un atome d'hydrogène ou un radical méthyle et Q représente un radical alkyle ou aryle.

Dans la formule (II) ci-dessus, le symbole Q sert à désigner de préférence un radical alkyle inférieur, méthyle, éthyle, propyle ou butyle par exemple, ou un radical aryle tel que phényle ou p-tolyle par exemple.

EXALTONE® et muscone sont toutes deux des cétones macrocycliques fort appréciées dans l'art de la parfumerie por leur odeur musquée. Connues depuis fort longtemps déjà, elles ont suscité de tout temps l'intérêt des chercheurs, vu le grand nombre de synthèses publiées dans la littérature scientifique [voir par exemple J. Chem. Soc. *1964*, 4154; Tetrahedron *20*, 2601 (1964); Helv. Chim. Acta *50*, 705 (1967); Helv. Chim. Acta *50*, 708 (1967)].

Toutes ces méthodes cependant ne s'appliquent qu'avec difficultés à l'échelle industrielle, soit à cause de leur complexité, soit à cause du faible rendement de certaines étapes clés.

L'une des méthodes synthétiques connues à ce jour [voir Helv. Chim. Acta *50*, 705 (1967)] fait appel au composé de formule

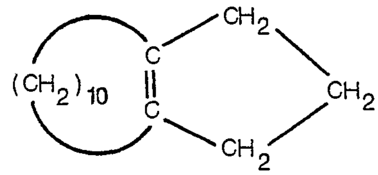

(Ia)

(R = H dans la formule I) à titre de produit intermédiaire dans la synthèse d'EXALTONE® et à son homologue méthylé, de formule

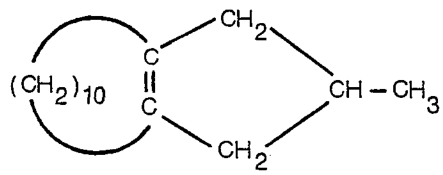

(Ib)

(R = $CH_3$ dans la formule I) pour la préparation de muscone. Ces composés s'obtiennent à partir de cyclododécanone, après toute une série d'étapes réactionnelles faisant notamment intervenir condensation, cyclisation, hydrogénation et déshydrogénation. Il s'en suit un rendement global relativement bas et de ce fait peu d'intérêt du point de vue industriel.

K. Biemann et al. [voir: J. Am. Chem. Soc. *79,* 5558—5564 (1957)] ont décrit un procédé pour l'obtention du composé (Ia), lequel utilise également la cyclododécanone comme produit de départ. Un tel procédé se heurte cependant à une difficulté majeure car la réduction de l'intermédiaire préconisé, à savoir la bicyclo[10.3.0]-(12)-pentadécène-13-one, conduit à la formation d'un mélange qui, à côté de l'olefine bicyclique désirée, contient une quantité prépondérante ($\geqslant$70%) de l'oléfine isomérique, ou bicyclo[10.3.0]-12-pentadécène.

L'invention a le mérite de proposer un procédé nouveau, original, permettant d'obtenir plus avantageusement lesdits composés de formule (I).

Selon l'invention, les composés (II) sont soumis à un traitement au moyen d'une base forte, en présence ou non d'un solvant ou mélange de solvants organiques, pour aboutir au composé bicyclique polyinsaturé (III) correspondant.

Du point de vue formel, on peut distinguer une phase initiale de condensation suivie d'une phase d'élimination, toutes deux effectuées sous l'action d'une base forte. Condensation et élimination d'eau subséquente conduisent à un produit intermédiaire de formule

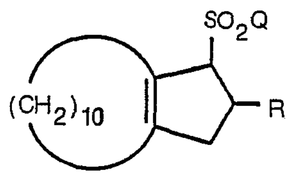

(IV)

(R et Q sont définis comme précédemment) que l'on peut isoler, si désiré, du mélange de réaction. Ledit composé (IV), sous l'action d'une base identique ou différente de celle conduisant au composé ci-dessus, subit une seconde élimination, aboutissant finalement au composé bicyclique polyinsaturé (III) défini précédemment. Pour des motifs d'ordre pratique et économique, les phases exposées ci-dessus sont de préférence réunies en une seule étape réactionnelle.

A titre de base forte, on peut utiliser, selon l'invention, une base forte organique telle un alcoolate de métal alcalin, le tert-butylate de potassium, le tertamylate ou le méthylate de sodium par exemple, ou encore un hydrure ou amidure de sodium ou potassium par exemple. Selon les cas, on peut même utiliser un mélange de deux bases organiques, méthylate de sodium et tert-butylate de potassium par exemple. On peut également utiliser une base forte minérale, l'hydroxyde de potassium par exemple.

On utilise ladite base en excès par rapport au composé (II), le plus généralement a raison d'au moins 2 a 2,5 équivalents de base pour 1 équivalent dudit composé (II). Selon la nature de la base, on pourra l'utiliser à raison de 6, voire 10 équivalents environ pour 1 équivalent de composé (II).

Comme indiqué ci-dessus, ledit traitement peut être effectué sans apport de solvant, par simple traitement thermique du composé (II) en présence de la base choisie. C'est ainsi, par exemple, que la 2-(2-méthyl-3-phénylsulfonyl-prop-1-yl)-cyclododécanone peut être convertie en composé (III) correspondant par chauffage à 160°C environ en présence de tert-butylate de potassium.

Lorsque ledit traitement s'effectue en présence d'un solvant ou mélange de solvants organiques, on peut avantageusement utiliser un hydrocarbure aromatique, toluène ou xylène par exemple, un alcool aliphatique, un éther tel que le tétrahydrofuranne, le dioxanne ou le diméthoxy-éthane par

exemple, ou encore le diméthyl-sulfoxyde, l'éthylène-diamine ou l'hexaméthyl-phosphore-triamide (HMPT) par exemple. Selon la nature du solvant ou mélange de solvants choisi, la température de réaction pourra varier entre 60 et 160°C environ, de préférence entre 100 et 120°C environ. Elle est le plus généralement voisine de l'ébullition du solvant ou mélange de solvants choisi.

Les composés bicycliques polyinsaturés (III) ainsi obtenus peuvent être ensuite convertis, selon l'invention, en composé (I) par hydrogénation en présence d'un catalyseur métallique. Ladite hydrogénation s'effectue le plus généralement à pression ambiante, en présence d'un catalyseur d'hydrogénation apte à provoquer également l'isomérisation d'une double liaison: on utilise de préférence le palladium sur charbon.

Ladite hydrogénation peut s'effectuer en présence ou non d'un solvant organique inerte, dans un tel cas en présence d'un hydrocarbure aliphatique, l'éther de pétrole par exemple, ou aromatique, benzène, toluène, toluène ou xylène par exemple, ou aromatique, benzène, toluène ou xylène par exemple, ou un alcool ou un éther, en fait, tout solvant ou mélange de solvants organiques apte à solubiliser le composé (III). Ladite hydrogénation s'effectue en outre à une température le plus généralement comprise entre environ 20 et 120°C, les meilleurs rendements en produit final étant cependant obtenus lorsque ladite hydrogénation s'effectue à des températures comprises entre environ 75 et 115°C.

Les composés (II), utilisés à titre de produits de départ dans le procédé de l'invention, sont des composés nouveaux. Ils s'obtiennent aisément à partir de 2-allyl-cyclododécanone, respectivement 2-méthallyl-cyclododécanone, après addition radicalaire du thiol approprié et oxydation subséquente. Chacune des étapes réactionnelles ci-dessus fait appel à des techniques connues; elles sont décrites plus en détail dans les exemples illustrant l'invention. Le schéma ci-après résume la préparation desdits composés (II):

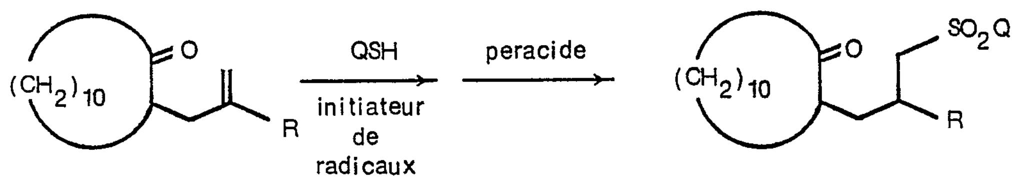


(II)

Dans ledit schéma, les symboles R et Q sont définis comme indiqué précédemment. Dans les exemples ci-dessous, les températures sont indiquées en degrés centigrades et les abréviations possèdent le sens usuel dans l'art.

Exemple 1

*Bicyclo[10.3.0]pentadéca-1,12-diène* (produit intermédiaire non isolé)

a) méthode avec tert-butOK

0,728 g (2 mmole) de 2-(3-phénylsulfonyl-prop-1-yl)cyclododécanone et 0,560 g (5 mmole) de tert-butylate de potassium en solution dans 8 ml de toluène ont été chauffés durant 1 h à reflux dans un réacteur muni d'un séparateur d'eau. Après refroidissement du mélange réactionnel à 10°, addition de $H_2O$ (15 ml), extraction à l'éther (2 x 15 ml), lavage des extraits organiques avec une solution saturée de NaCl (15 ml), puis $H_2O$, séchage sur $Na_2SO_4$, évaporation et finalement distillation (3 Pascals — température du bain 140°), on a isolé 0,340 g (rendement 83%) du produit désiré.

IR: 2910, 1460, 1440, 925, 915, 845 $cm^{-1}$

RMN (90 MHz): 1,02—1,76 (14H, m); 1,95—2,68 (8H, m); 5,47 (1H, t, J=7,5 Hz); 5,72 (1H, large s) $\delta$ ppm

SM: $M^+ + 1 = 205(15)$, $M^+ = 204(84)$; m/e = 175(8), 161(34), 148(60), 147(78), 134(42), 133(100), 119(62), 106(42), 105(57), 93(47), 91(81), 79(70), 67(58).

b) méthode avec $NaOHC_3$/tert-butOK

29,0 ml d'une solution de méthylate de sodium à 30% dans l'alcool méthylique (0,15 mole) ont été ajoutés goutte à goutte à un mélange de 36,4 g (0,10 mole) de 2-(3-phénylsulfonyl-prop-1-yl)-cyclododécanone et 200 ml de toluène maintenu à reflux dans un réacteur muni d'un séparateur d'eau (durée de l'addition: environ 1 h). Après cette première addition, 14,6 g (0,13 mole) de tert-butylate de potassium ont encore été ajoutés au mélange de réaction et le chauffage à reflux poursuivi durant 4 h. Après extraction et distillation comme indiqué plus haut, on a recueilli 17,1 g (rendement 84%) du produit désiré

c) méthode avec $NaOCH_3$

2,37 ml d'une solution de méthylate de sodium à 30% dans l'alcool méthylique (10,2 mmole) ont été ajoutés goutte à goutte à 0,728 g (2,0 mmole) de 2-(3-phénylsunfonyl-prop-1-yl)-cyclododécanone dans 10 ml de toluène, comme indiqué sous lettre b). Après 90 minutes de chauffage à

reflux 0,57 ml (8,0 mmole) de diméthyl-sulfoxyde (DMSO) ont été ajoutés au mélange de réaction et le chauffage à reflux poursuivi durant 4 h. Après extraction et distillation comme indiqué plus haut, on a isolé 0,310 g (rendement 76%) du produit désiré.

d) méthode avec KOH

Une suspension de 10,92 g (0,03 mole) de 2-(3-phénylsulfonyl-prop-1-yl)-cyclododécanone et 11,0 g (0,197 mole) de KOH fraîchement pulvérisé dans 40 ml de toluène a été chauffée durant 30 minutes à reflux, comme indiqué sous lettre b). Après addition de 6 ml (0,085 mole) de DMSO, le chauffage à reflux a été poursuivi pendant 12 h. Après extraction et distillation comme indiqué précédemment on a recueilli 5,32 g (rendement 87%) du produit désiré.

En ajoutant 13,5 g (0,24 mole) de KOH au lieu des 11 g sus-mentionnés, on a pu réduire le temps de chauffage en présence de DMSO à 30 minutes.

e) méthode avec KOH/Ethanol

0,728 g (2 mmole) de 2-(3-phénylsulfonyl-prop-1-yl)-cyclododécanone et 0,730 g )13 mmole) de KOH en solution dans 10 ml d'éthanol ont été chauffés durant 1 h à reflux. Après addition de 2 ml de DMSO, le melange de réaction a été porté à 120° et l'éthanol éliminé par distillation. Le mélange résultant a encore été chauffé à environ 110° durant 12 h, puis traité comme indiqué plus haut pour finalement donner 0,29 g (rendement 71%) du produit désiré.

f) méthode avec NaH

0,144 g (6 mmole) d'hydrure de sodium et 10 ml de DMSO ont été chauffés durant 30 min. à 80°. Une fois l'évolution d'hydrogène terminée, 0,728 g (2 mmole) de 2-(3-phénylsulfonyl-prop-1-yl)-cyclododécanone ont été ajoutés au mélange de réaction et le tout a été porté durant 4 h à 100°. Après les traitements usuels d'extraction et purification, on a finalement isolé 0,326 g (rendement 80%) du produit désiré.

g) méthode avec NaH/Ethylène-diamine

0,728 g (2 mmole) de 2-(3-phénylsulfonyl-propyl-1-yl)-cyclododécanone, 0,144 g (6 mmole) d'hydrure de sodium et 10 ml d'éthylène-diamine ont été premièrement chauffés durant 4 h à environ 100°. Après les traitements usuels (voir plus haut), on a isolé le produit désiré avec un rendement de 75%.

h) méthode avec tert-amylate de Na

18,2 g (0,05 mole) de 2-(3-phénylsulfonyl-prop-1-yl)-cyclododécanone, 33,0 g (0,30 mole) de tert-amylate de sodium et 100 ml de toluène ont été traités comme indiqué sous lettre a). Après addition de 15 ml de DMSO, reprise du chauffage durant 4 h à environ 110°, extraction et distillation comme indiqué précédemment, on a recueilli 7,75 g (rendement 76%) du produit désire.

Exemple 2

*Bicyclo[10.3.0]pentadéca-1,12-diène* (produit intermédiaire isolé)

i) 0,728 g (2,0 mmole) de 2-(3-phénylsulfonyl-prop-1-yl)-cyclododécanone et 0,336 g (3,0 mmole) de tert-butylate de potassium en solution dans 8 ml de toluène ont été chauffés durant 1 h à 50°. Après refroidissement, addition de $H_2O$ (15 ml), extraction à l'éther (2 × 15 ml), lavages et séchage comme indiqué plus haut, on a recueilli, après évaporation, 0,673 g d'un produit solide. Après recristallisation dans un mélange éther/éther de pétrole, on a finalement isolé 0,598 g (rendement 86%) d'un composé de formule

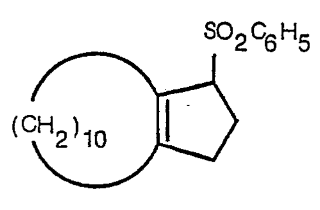

Ce dernier a été caractérisé comme suit: F. 94—99°

IR: 2920, 1460, 1440, 1300, 1285, 1125, 1080 $cm^{-1}$

RMN (90 MHz): 0,95—1,95 (18H, m); 2,00—2,70 (6H, m); 4,20 (1H, d, J=7 Hz); 7,40—7,95 (5H, m) $\delta$ ppm

SM: m/e = 219(15), 218(8), 147(7), 133(7), 119(14), 107(17), 105(15), 95(35), 91(24), 81(21), 77(19), 55(19), 44(30), 40(100).

Des résultats similaires ont été obtenus en remplaçant, dans l'exemple ci-dessus, le tert-butylate de potassium par une quantité équivalente de méthylate de sodium.

Ledit composé a également été obtenu en traitant 1 équivalent de 2-(3-phénylsulfonyl-prop-1-yl)-cyclododécanone au moyen de 7 équivalents de KOH fraîchement pulvérisé (rendement 88%).

ii) le composé ainsi obtenu a ensuite été cnverti en bicyclo[10.3.0]pentadeca-1,12-diène désiré après chauffage à environ 110° dans du toluène, en présence de tert-butylate de potassium (1 équivalent) durant 1 h; rendement 82%. Le produit ainsi préparé est identique à celui obtenu à l'Exemple 1.

La 2-(3-phénylsulfonyl-prop-1-yl)-cyclododécanone utilisée ci-dessus (Exemples 1 et 2) comme produit de départ, a été préparée comme suit: 222 g (1 mole) de 2-allyl-cyclododécanone — Helv. *54,* 2889 (1971) — en mélange avec 132 g (1.2 mole) de thiophénol et 3,0 g (0,018 mmole) d'$\alpha,\alpha'$-azoisobutyronitrile ont été chauffés durant 10 h à 100°, période au cours de laquelle 6,0 g supplémentaires (0,037 mole) d'$\alpha,\alpha'$-azoisobutyronitrile ont été ajoutés au mélange réactionnel, par petites portions. Après dissolution dans un excès de trichloroéthane et refroidissement dans un bain de glace, 376 ml (2,6 mole) d'acide peracétique à 40% y ont été incorporés sous agitation, de façon à maintenir la température du mélange aux environs de 25—30°. Après élimination de l'excès de peroxydes au moyen d'une solution aqueuse de $NaHSO_3$, on a ajouté une solution de NaOH à 10% dans $H_2O$ jusqu'à obtention d'un pH de 8—9 (température environ 10°). Après extraction avec $CH_2Cl_2$ (2 × 300 ml), lavages succesifs avec $H_2O$ et $H_2O$ saturée en NaCl, séchage sur $Na_2SO_4$ et évaporation, on a isolé un premier résidu solide. Après recristallisation dans un mélange $CH_2Cl_2$/éther/éther de pétrole, on a finalement recueilli 332 g (rendement 91%) du produit désiré.

F. 97—102°
IR: 2950, 1700, 1465, 1445, 1300, 1145, 1080, 790 $cm^{-1}$
RMN (60 MHz): 1,00—2,00 (22H, m); 2,30—2,75 (3H, m); 2,90—3,25 (2H, m); 7,50—8,02 (5H, m) $\delta$ ppm
SM: $M^+$ = 364(30); m/e = 254(8), 240(15), 223(12), 143(32), 98(41), 55(100), 41(59).

Exemple 3

*14-Méthyl-bicyclo[10.3.0]pentadéca-1,12-diène* (produit intermédiaire non isolé)

Une suspension de 11,35 g (0,03 mole) de 2-(2-méthyl-3-phénylsulfonyl-prop-1-yl)-cyclododécanone et 11,0 g (0,197 mole) de KOH fraîchement pulvérisé dans 30 ml de toluène a été premièrement chauffée durant 30 minutes à reflux, dans un réacteur muni d'un séparateur d'eau. Après addition de 6 ml de DMSO, la mélange de réaction a encore été chauffé durant 12 h à environ 110°. Après les traitements d'extraction indiqués précédemment et distillation (7 Pascals — température du bain 150°), on a recueilli 5,61 g (rendement 86%) du produit désiré.

IR: 2920, 1460, 1440, 835 $cm^{-1}$
RMN (90 MHz): 1,00 (3H, d, J=7 Hz); 1,08—1,73 (14H, m); 1,80—2,40 (5H, m); 2,55—2,91 (2H, m); 5,40 (1H, t, J=7,5 Hz); 5,60 (1H, large s) × ppm
SM: $M^+$ = 218(93); m/e = 203(80), 175(30),161(70), 147(100), 133(92), 112(68), 107(60), 106(44), 105(68), 94(55), 93(72), 91(75), 79(55), 67(32), 55(38).

Exemple 4

*14-Méthyl-bicyclo[10.3.0]pentadéca-1,12-diène* (produit intermédiaire isolé)

i) 0,58 ml d'une solution de méthylate de sodium à 30% dans le méthanol (3 mmole) ont été ajoutés à 0,756 g (2 mmole) de 2-(2-méthyl-3-phénylsulfonyl-prop-1-yl)-cyclododécanone en solution dans 8 ml de toluène et le mélange résultant chauffé durant 1 h à reflux dans un réacteur muni d'un séparateur d'eau. Après les traitements usuels d'extraction à l'éther et distillation (1 Pascal — température du bain 200°), on a isolé 0,627 g (rendement 87%) d'un composé de formule

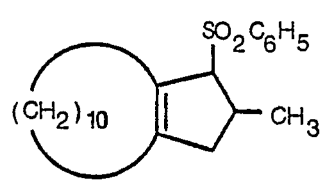

Ce dernier a éte identifié comme suit:
IR: 2945, 1470, 1445, 1300, 1140, 1080 $cm^{-1}$
RMN (90 MHz): 0,90—1,80 (22H, m); 2,20—3,00 (4H, m); 3,78 (1H, large s); 7,35—7,96 (5H, m) $\delta$ ppm
SM = m/e = 220(9), 219(48), 149(9), 147(12), 133(13), 123(13), 119(24), 109(22), 107(33), 105(28), 95(38), 94(71), 93(30), 91(45), 81(62), 79(29), 77(29), 69(44), 67(31), 57(36), 55(50), 44(44), 43(40), 41(56), 40(100).

ii) le composé ainsi obtenu a ensuite été converti en 14-méthyl-bicyclo[10.3.0]pentadéca-1,12-diène désiré après chauffage à environ 110° dans du toluène, en présence d'un équivalent de tert-butylate de potassium et traitements usuels subséquents (voir Exemple 3).

La 2-(2-méthyl-3-phénylsulfonyl-prop-1-yl)-cyclododécanone, utilisée ci-dessus (Exemples 3 et 4) à titre de produit de départ, a été préparée comme suit: un mélange de 236 g (1 mole) de 2-méthallyl-cyclododécanone — Chem. Com. *1976,* 1021—132 g (1,2 mole) de thiophénol et 3,0 g (0,018 mole) d'$\alpha,\alpha'$-azoisobutyronitrile a été chauffé durant 32 h à 115°. Au cours de cette période, 10 g (0,061 mole) supplémentaires d'$\alpha,\alpha'$-azoisobutyronitrile ont encore été ajoutés au mélange réactionnel, par petites portions. Le mélange résultant, repris dans un excès de trichloroéthane, a ensuite été oxydé au moyen de 2,6-mole (376 ml) d'acide peracétique à 40% selon le procédé de l'Exemple 2. Après les traitements de réduction, neutralisation et extraction mentionnés précédemment, on a recueilli, après recristallisation dans un mélange $CH_2Cl_2$/éther/éther de pétrole, 325 g (rendement 86%) du produit désiré.

F. 98—103°

IR: 2950, 1705, 1470, 1450, 1305, 1145, 1085 $cm^{-1}$

RMN (60 MHz): 0,90—2,20 (24H, m); 2,30—2,75 (3H, m); 3,00 (2H, d, J=5 Hz); 7,50—8,02 (5H, m) $\delta$ ppm

SM: $M^+ = 378(14)$; m/e = 255(20), 237(22), 143(15), 98(24), 95(32), 83(52), 81(49), 77(50), 69(36), 67(32), 55(100), 43(41), 41(83).

Exemple 5

*Bicyclo[10.3.0]pentadéc-1(12)-ène*

6,06 g de bicyclo[10.3.0]pentadéca-1,12-diène brut tel qu'obtenu selon le procédé de l'Exemple 1, en solution dans 40 ml de toluène, ont été hydrogénés à 100° et pression atmosphérique en présence de 0,6 g de palladium à 10% sur charbon. Après 60 minutes d'hydrogénation, le mélange de réaction a été refroidi, filtré sur CELITE et finalement distillé (3 Pascals — température du bain 140°) pour donner 5,2 g (rendement 84%) de bicyclo[10.3.0]pentadéc-1(12)-ène ayant une pureté de 93% selon l'analyse par chromatographie en phase gazeuse (CARBOWAX 10% — 1,6 m — 140°) et spectrométrie de masse.

Le produit ainsi obtenu est en tous points identique à un échantillon préparé selon J. Amer. Chem. Soc. *79,* 5558 (1957).

Des résultats similaires ont été obtenus en effectuant ladite hydrogénation dans le xylène à 115°, respectivement dans l'éthanol à 75°.

Exemple 6

*14-Méthyl-bicyclo[10.3.0]pentadéc-1(12)-ène*

6,48 g de 14-méthyl-bicyclo[10.3.0]pentadéca-1,12-diène brut tel qu'obtenu selon le procédé de l'Exemple 3 en solution dans 40 ml de toluène, ont été hydrogénés à 100° et pression atmosphérique en présence de 0,65 g de palladium à 10% sur charbon (période d'hydrogénation: 60 minutes). Après les traitements usuels subséquents décrits à l'Exemple 5, on a isole 5,69 g (rendement 86%) de 14-méthyl-bicyclo[10.3.0]pentadéc-1(12)ène ayant une pureté de 95% selon l'analyse par chromatographie en phase gazeuse (CARBOWAX 10% — 1,6 m — 140°) et spectrométrie de masse.

Le produit ainsi obtenu est en tous points identique au produit obtenu selon Chem. Abstr. *70,* 88108 v (1970).

Exemple 7

*14-Méthyl-bicyclo[10.3.0]pentadéca-1,12-diène*

0,756 g (2,0 mmole) de 2-(2-méthyl-3-phénylsulfonyl-prop-1-yl)-cyclododécanone et 0,672 g (6,0 mmole) de tert-butylate de potassium ont été portés à 160° sous 3 Pascals et le 14-méthyl-bicyclo[10.3.0]pentadéca-1,12-diène désiré distillé au fur et à mesure de sa formation (temps de réaction environ 30 minutes). On a ainsi recueilli 0,216 g (rendement environ 50%) du produit désiré.

Exemple 8

*Bicyclo[10.3.0]pentadéca-1,12-dièbe*

10 g (0.029 mole) de 2-(3-n-butylsulfonyl-prop-1-yl)-cyclododécanone en suspension dans 40 ml de toluène ont été chauffés durant 30 minutes à reflux, en présence de 11 g (0,197 mole) de KOH pulvérisé (voir Exemple 1, lettre b). Après addition de 6 ml de DMSO, reprise du chauffage à reflux pendant 12 h et finalement extraction et distillation comme indiqué plus haut, on a isolé le produit désiré avec un rendement de 84%.

La 2-(3-n-butylsulfonyl-prop-1-yl)-cyclododécanone utilisée ci-dessus comme produit de départ a été obtenue à partir de 2-allyl-cyclododécanone et de n-butylthiol, après chauffage à 95° durant 10 h en présence d'$\alpha,\alpha'$-azoisobutyronitrile (voir également Exemple 2).

Rendement 88%; F. 91,5°
IR: 2950, 1700, 1470, 1300, 1140 $cm^{-1}$
RMN: 0,85—2,10 (29H, m); 2,25—3,15 (7H, m) $\delta$ ppm
SM: $M^+ = 344(5)$; m/e = 261(4), 233(9), 220(20), 123(48), 98(49), 83(40), 69(60), 55(100), 41(83).

**Revendications**

1. Composés de formule

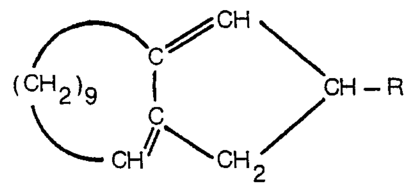

(III)

dans laquelle le symbole R représente un atome d'hydrogène ou un radical méthyle.

2. Procédé pour la préparation d'un composé de formule (III) telle que définie à la revendication 1, caractérisé en ce qu'on traite au moyen d'une base forte un composé de formule

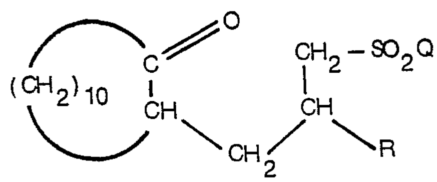

(II)

dans laquelle le symbole R est défini comme pour la formule (III) et Q représente un radical alkyle, de préférence un radical alkyle inférieur tel que méthyle, éthyle, propyle ou butyle par exemple, ou un radical aryle tel que phényle ou p-tolyle par exemple.

3. Utilisation d'un composé de formule (III) telle que définie à la revendication 1 à titre de produit de départ pour la préparation d'un composé de formule

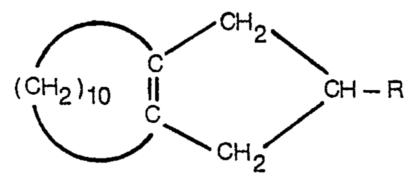

(I)

dans laquelle le symbole R représente un atome d'hydrogène ou un radical méthyle, caractérisée en ce qu'on hydrogène ledit composé de formule (III) en présence d'un catalyseur métallique.

4. Composés de formule

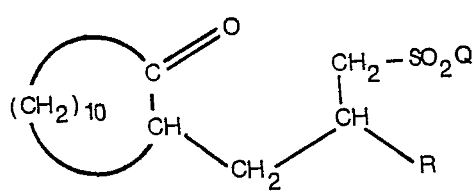

(II) (II)

dans laquelle le symbole R représente un atome d'hydrogène ou un radical méthyle et Q représente un radical alkyle, de préférence un radical alkyle inférieur tel que méthyle, éthyle, propyle ou butyle par exemple, ou un radical aryle tel que phényle ou p-tolyle par exemple.

**Claims**

1. Compounds of formula

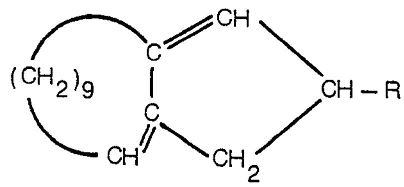

(III)

wherein symbol R represents a hydrogen atom or a methyl radical.

2. Process for the preparation of a compound of formula (III) as defined in claim 1, characterized in that a compound of formula

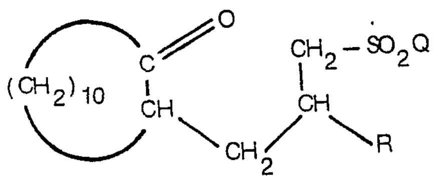

(II)

wherein symbol R is defined as for formula (III) and Q represents an alkyl radical, preferably a lower alkyl radical such as methyl, ethyl, propyl or butyl for example, or an aryl radical such as phenyl or p-tolyl for example, is treated with a strong base.

3. Utilization of a compound of formula (III) as defined in claim 1 as starting material for the preparation of a compound of formula

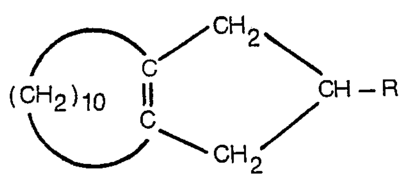

(I)

wherein symbol R represents a hydrogen atom or a methyl radical, characterized in that said compound of formula (III) is hydrogenated in the presence of a metal catalyst.

4. Compounds of formula

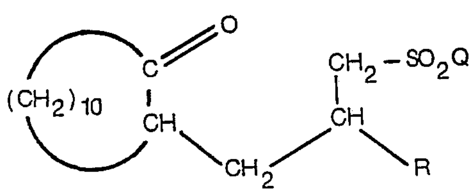

(II)

wherein symbol R represents a hydrogen atom or a methyl radical and Q represents an alkyl radical, preferably a lower alkyl radical such as methyl, ethyl, propyl, or butyl for example, or an aryl radical such as phenyl or p-toluyl for example.

**Patentansprüche**

1. Verbindungen der Formel

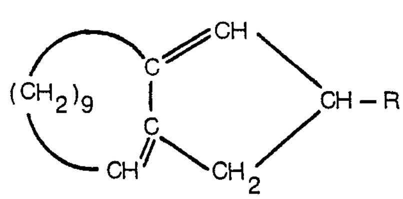

(III)

worin das Symbol R ein Wasserstoffatom oder einen Methylrest darstellt.

2. Verfahren zur Herstellung einer Verbindung der Formel (III) gemäss Anspruch 1, dadurch gekennzeichnet, dass man eine Verbindung der Formel

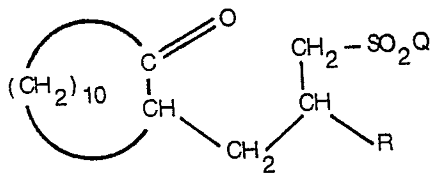 (II)

worin das Symbol R wie für formel (III) definiert ist und Q einen Alkylrest, vorzugsweise einen niedrigen Alkylrest, wie z.B. Methyl, Ethyl, Propyl oder Butyl, oder einen Arylrest, wie z.B. Phenyl oder p-Tolyl, darstellt, mittels einer starken Base umsetzt.

3. Verwendung einer Verbindung der Formel (III) gemäss Anspruch 1 als Ausgangsmaterial zur Herstellung einer Verbindung der Formel

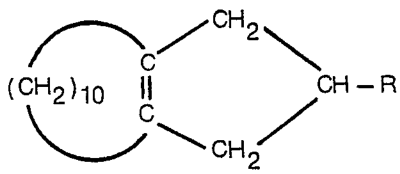 (I)

worin das Symbol R ein Wasserstoffatom oder einen Methylrest darstellt, dadurch gekennzeichnet, dass man die erwähnte Verbindung der Formel (III) in Anwesenheit eines Metallkatalysators hydriert.

4. Verbindungen der Formel

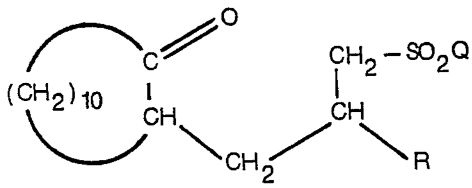 (II)

worin das Symbol R ein Wasserstoffatom oder einen Methylrest darstellt und Q einen Alkylrest, vorzugsweise einen niedrigen Alkylrest, wie z.B. Methyl, Ethyl, Propyl oder Butyl, oder einen Arylrest, wie z.B. Phenyl oder p-Tolyl, darstellt.